# FASCICULE DE BREVET EUROPEEN

(11) **EP 1 703 914 B1**
(45) Date de publication et mention de la délivrance du brevet: **16.04.2008**
(21) Numéro de dépôt: 05717409.6
(22) Date de dépôt: 13.01.2005
(51) Int. Cl.: A61K 35/68, C12N 1/11, A61K 39/002, A61P 33/02

(54) **SOUCHES VACCINALES D'APICOMPLEXES DE LA FAMILLE DES SARCOCYSTIDAE**
APIKOMPLEX-VAKZINE-STÄMME EINER FAMILIE VON SARCOCYSTIDAE
APICOMPLEX VACCINE STRAINS OF A FAMILY OF SARCOCYSTIDAE

(30) Priorité: 13.01.2004 FR 0400260
(43) Date de publication de la demande: 27.09.2006
(73) Titulaire: INSTITUT NATIONAL DE LA RECHERCHE AGRONOMIQUE, 75007 Paris (FR); CENTRE NATIONAL DE LA RECHERCHE SCIENTIFIQUE, 75016 Paris Cédex (FR); Universite Francois Rabelais, 37041 Tours Cedex 1 (FR)
(72) Inventeur: DUBREMETZ, Jean-François, F-34090 MONTPELLIER (FR); BOUT, Daniel, F-37390 CERELLES (FR); LEBRUN, Maryse, F-34830 JACOU (FR); SOÊTE, Martine, F-62000 ARRAS (FR); CEREDE, Odile, F-34000 MONTPELLIER (FR)
(74) Mandataire: Vialle-Presles, Marie José
(86) Numéro de dépôt international: PCT/FR2005/000074
(87) Numéro de publication internationale: WO 2005/072754

(56) Documents cités:
- FR-A- 2 805 466
- BUXTON D ET AL: "A COMMERCIAL VACCINE FOR OVINE TOXOPLASMOSIS" PARASITOLOGY, CAMBRIDGE UNIVERSITY PRESS, LONDON, GB, vol. 110, 1995, pages S11-S16, XP009033480 ISSN: 0031-1820
- CHARTIER C ET AL: "EFFICACITE VACCINALE DE LA SOUCHE S48 DE TOXOPLASMA GONDII VIS-A-VIS D'UNE INFECTION EXPERIMENTALE CHEZ LA CHEVRE" ANNALES DE MEDICINE VETERINAIRE, FACULTE DE MEDECINE VETERINAIRE, LIEGE, BE, vol. 145, no. 3, 2001, pages 202-209, XP009033482 ISSN: 0003-4118
- MONTOYA J G ET AL: "Toxoplasmosis" LANCET THE, LANCET LIMITED. LONDON, GB, vol. 363, no. 9425, 12 juin 2004 (2004-06-12), pages 1965-1976, XP004515491 ISSN: 0140-6736
- FOURMAUX M N ET AL: "The MIC1 microneme protein of Toxoplasma gondii contains a duplicated receptor-like domain and binds to host cell surface" MOLECULAR AND BIOCHEMICAL PARASITOLOGY, ELSEVIER SCIENCE PUBLISHERS, AMSTERDAM, NL, vol. 83, 1996, pages 201-210, XP002198882 ISSN: 0166-6851
- BOURGUIN I ET AL: "Murine dendritic cells pulsed in vitro with Toxoplasma gondii antigens induce protective immunity in vivo" INFECTION AND IMMUNITY, AMERICAN SOCIETY FOR MICROBIOLOGY. WASHINGTON, US, vol. 66, no. 10, octobre 1998 (1998-10), pages 4867-4874, XP002225885 ISSN: 0019-9567
- DUBREMETZ J F: "HOST CELL INVASION BY TOXOPLASMA GONDII" TRENDS IN MICROBIOLOGY, ELSEVIER SCIENCE LTD., KIDLINGTON, GB, vol. 6, no. 1, janvier 1998 (1998-01), pages 37-40, XP000960333 ISSN: 0966-842X
- BHOPALE G., ET AL: "Development of a vaccine for toxoplasmosis: current status" MICROBES AND INFECTION, vol. 5, 2003, pages 457-462, XP002289505

### SEQUENCE LISTING

<110> INSTITUT NATIONAL DE LA RECHERCHE AGRONOMIQUE CENTRE NATIONAL DE LA RECHERCHE SCIENTIFIQUE UNIVERSITE FRANCOIS RABELAIS
   DUBREMETZ, Jean-François
   BOUT, Daniel
   LEBRUN, Maryse
   SOÊTE, Martine
   CEREDE, Odile
<120> SOUCHES VACCINALES D'APICOMPLEXES DE LA FAMILLE DES *SARCOCYSTIDAE*
<130> MJPbv539/120
<150> FR04 00260
   <151> 2004-01-13
<160> 6
<170> PatentIn version 3.1
<210> 1
   <211> 27
   <212> DNA
   <213> Artificial sequence
<220>
   <223> Amorce PCR: ML9
<400> 1
   gtgtaagctt cagcgagtct ctgagag 27
<210> 2
   <211> 32
   <212> DNA
   <213> Artificial sequence
<220>
   <223> Amorce PCR: ML10
<400> 2
   ggggtaccga gctcatgagc agaagctgcc ag 32
<210> 3
   <211> 32
   <212> DNA
   <213> Artificial sequence
<220>
   <223> Amorce PCR: ML23
<400> 3
   ctgaattcag atcttaccag tgttggacaa gg 32
<210> 4
   <211> 31
   <212> DNA
   <213> Artificial sequence
<220>
   <223> Amorce PCR: ML24
<400> 4
   ggggtacccc ttgctaggta accactcgtg c 31
<210> 5
   <211> 36
   <212> DNA
   <213> Artificial sequence
<220>
   <223> Amorce PCR: ML11
<400> 5
   gcacaattga gatctaaaat gcgaggcggg acgtcc 36
<210> 6
   <211> 41
   <212> DNA
   <213> Artificial sequence
<220>
   <223> Amorce PCR: ML15
<400> 6
   tgctatgcat tcctaggctg cttaattttc tcacacgtca c 41

## Description

La présente invention est relative à des souches mutantes atténuées d'Apicomplexes de la famille des *Sarcocystidae,* tels que *Toxoplasma* et *Neospora,* et à leurs utilisations vaccinales.

Le phylum des Apicomplexes (embranchement *Apicomplexa),* regroupe un grand nombre de parasites majoritairement intracellulaires. Ces parasites sont responsables de maladies telles que la toxoplasmose, la malaria, la néosporose, la coccidiose et la cryptosporidiose. Ils ont en commun un processus spécifique d'invasion de cellules hôtes en plusieurs étapes, conduisant à la formation d'une vacuole parasitophore dans laquelle le parasite se développe (MENARD et al., Cell Microbiol. 3 : 63-73, 2001 ; SOLDATI et al., Int. J. Parasitol. 31 : 1293-1302, 2001).

*Toxoplasma gondii* est un parasite protozoaire intracellulaire obligatoire, responsable de la toxoplasmose humaine et animale. Il appartient à la famille des *Sarcocystidae* qui regroupe également d'autres pathogènes majeurs de l'homme et de l'animal, tels que *Neospora* ou *Sarcocystis* (LEVINE, The Protozoan Phylum Apicomplexa. Vol.1, CRC Press, Boca Raton, FL, p.203, 1988 ; TENTER et al., Int. J. Parasitol. 32(5) : 595-616, 2002). Son cycle de vie présente deux aspects distincts : un cycle « asexué » chez un hôte intermédiaire, tel que l'homme, la souris, les ovins et les porcins, conduisant à la production de tachyzoïtes puis de kystes contenant des bradyzoïtes ; et un cycle « sexué » chez le chat conduisant à la production d'oocystes (contenant des sporozoïtes) éliminés dans les fèces.

La toxoplasmose animale pose un problème économique important dans le domaine de l'élevage agricole. Elle atteint tous les animaux de rente. La transmission à ces animaux se fait par l'ingestion d'oocystes, formes de résistance émises dans l'environnement par des chats infectés par un toxoplasme pathogène. Chez les ovins, les caprins et les porcs infectés pendant la gestation, elle provoque des avortements. Dans l'Union Européenne dont le cheptel ovin est estimé à 100 millions de têtes, 1 million d'agneaux sont perdus chaque année du fait d'avortements toxoplasmiques.

Par ailleurs la consommation de viande (surtout mouton et porc) infectée par la présence de bradyzoïtes, est la principale source des infections humaines. Contractée pendant la grossesse, la toxoplasmose est la 2^{ème} cause des malformations congénitales. En outre, au cours des vingt dernières années, ce parasite est apparu comme un pathogène opportuniste, à l'origine d'encéphalites chez les patients sidéens.

La mise au point de vaccins conférant une protection contre les parasitoses à *Apicomplexa,* fait l'objet de nombreuses recherches. Deux stratégies principales sont employées : 1) l'identification d'antigènes parasitaires capables d'induire une réponse immune protectrice, et l'incorporation de ces antigènes dans des compositions vaccinales ; 2) la sélection de souches de parasites atténuées. Par exemple, dans le cas de la toxoplasmose, il a été proposé d'utiliser différentes souches atténuées de *Toxoplasma gondii* (Brevet US 5,045,313; Brevet US 4,473,549 ; Demande de Brevet US 2002/0164754; Demande de Brevet GB 2 204 323), pour conférer aux mammifères une immunité anti-toxoplasmes.

A l'heure actuelle, un seul vaccin anti-toxoplasmose basé sur une souche atténuée est commercialisé. Il s'agit du vaccin TOXOVAX®, à base de tachyzoites vivants de la souche S48 de *Toxoplasma gondii.* La nature de la mutation responsable de l'atténuation de cette souche demeure inconnue.

Les étapes clefs de l'infection par les Apicomplexes, et en particulier par *Toxoplasma gondii,* sont l'attachement du parasite aux cellules-hôtes, suivi de l'invasion de celles-ci. L'appareil invasif des Apicomplexes implique l'exocytose séquentielle de deux types d'organelles sécrétoires : les micronèmes et les rhoptries.

De récentes études ont mis en évidence le rôle central des micronèmes dans la reconnaissance des cellules-hôtes, et l'adhésion à celles-ci. Les protéines des micronèmes, désignées sous l'appellation générique « MICs » contiennent des modules homologues aux domaines d'adhésion de protéines d'eucaryotes supérieurs (TOMLEY et SOLDATI, Trends Parasitol. 17 : 81-88, 2001).

Une douzaine de protéines MICs sont actuellement connues chez *Toxoplasma gondii* (SOLDATI et al., Int. J. Parasitol. 31 : 1293-1302, 2001). Certaines d'entre elles sont des protéines transmembranaires, par exemple MIC2 de *Toxoplasma gondii,* dénommée TRAP chez *Plasmodium* (MATUSCHEWSKI et al., EMBO J. 21 : 1597-1606, 2002) ; les autres sont des protéines solubles qui sont ciblées vers les micronèmes et redistribuées à la surface du parasite au cours de l'invasion, en association avec les protéines transmembranaires.

Récemment, deux protéines solubles MIC1 et MIC3, capables de se lier à la surface de cellules hôtes, ont été caractérisées chez *T. gondii* (ACHBAROU et al., Mol. Biochem. Parasitol., 47, 223-233, 1991 ; FOURMAUX et al., Mol. Biochem. Parasitol. 83 : 201-210, 1996 ; GARCIA-REGUET et al., Cell. Microbiol. 2 : 353-364, 2002).

La protéine MIC1 contient un domaine dupliqué en tandem possédant une lointaine homologie avec le domaine de type TSP-1 de TRAP, et présente une spécificité de liaison au lactose (LOURENCO et al., Glycobiol. 11 : 541-547,2001).

La protéine MIC3 est un dimère de poids moléculaire apparent de 90 kDa formé de deux sous-unités de 38 kDa reliées par des ponts disulfures. MIC3 contient cinq domaines de type EGF dont deux sont chevauchants, et un domaine de type « domaine de liaison à la chitine », riche en ponts disulfures, et qui apparaît nécessaire pour la liaison à la surface de la cellule hôte (GARCIA-REGUET et al., 2000, précité; CEREDE et al., EMBO J. 21: 2526-2536,2002).

MIC1 et MIC3 s'associent à d'autres protéines MICs pour former deux complexes indépendants, MIC1/4/6 et MIC3/8. Les protéines transmembranaires MIC6 et MIC8 jouent le rôle de transporteurs pour cibler respectivement les protéines MIC1/4 et MIC3 vers les micronèmes. La protéine MIC1 est indispensable pour que le complexe MIC1/4/6 puisse quitter les compartiments précoces de la voie de sécrétion (REISS et al., J. Cell Biol. 152 : 563-578, 2001).

Il a récemment été montré que la protéine MIC3 de *Toxoplasma gondii* constitue un antigène vaccinal majeur, suscitant une réponse immunitaire humorale précoce et très forte (Demande PCT WO 01/64243).

Dans le but d'étudier le rôle de MIC1 et MIC3 dans la capacité d'invasion et la virulence de *Toxoplasma gondii,* les Inventeurs ont construit des souches mutantes de *T. gondii,* dans lesquelles l'une et/ou l'autre des adhésines MIC1 et MIC3 ont été inactivées.

Ils ont constaté que l'inactivation de MIC1 diminue d'environ 50% la capacité d'invasion des fibroblastes *in vitro,* alors que celle de MIC3 ne modifie pas cette capacité d'invasion ; l'inactivation simultanée des deux protéines ne modifie pas significativement la capacité d'invasion par rapport à l'inactivation de MIC1 seule. La virulence *in vivo* n'est que très peu affectée par l'inactivation isolée de MIC1 et MIC3 ; en revanche, elle est fortement diminuée par l'inactivation simultanée des deux protéines.

Les Inventeurs ont en outre constaté que malgré l'absence des antigènes majeurs que constituent MIC1 et MIC3, une souche double mutante de *Toxoplasma gondii* dans laquelle ces deux protéines sont inactivées permet d'obtenir une protection vaccinale efficace vis-à-vis de la toxoplasmose.

Ils ont ensuite entrepris d'étudier les caractéristiques infectieuses et protectrices de ladite souche sur l'animal, en particulier sur les souris et les brebis.

Ils ont ainsi mis en évidence que la vaccination par cette souche protège les animaux contre la formation de kystes cérébraux lors d'une ré-infection par une souche sauvage pathogène de *Toxoplasma gondii;* ce qui diminue considérablement la portée d'une infection par cette souche sauvage pathogène, le risque de passage transplacentaire dans le cas de femelles gestantes, et la possibilité de transmission par consommation de la viande des animaux vaccinés, et donc à terme permet de faire baisser la prévalence générale de l'infection.

La présente invention a donc pour objet une souche mutante d'un Apicomplexe de la famille des *Sarcocystidae,* comprenant une mutation inactivant l'adhésine MIC1 et une mutation inactivant l'adhésine MIC3.

Selon un mode de réalisation préféré de la présente invention, ledit *Sarcocystidae* est choisi parmi *Toxoplasma et Neospora.*

Selon une disposition préférée de ce mode de réalisation ladite souche mutante est une souche de toxoplasme, notamment de *Toxoplasma gondii.*

On entend ici par :
- «mutation inactivant l'adhésine MIC1 » toute mutation résultant dans l'absence d'expression de MIC1, ou dans l'expression d'une protéine MIC1 non-fonctionnelle, c'est-à-dire incapable de former un complexe avec les protéines MIC4 et MIC6, ou incapable de lier le lactose.
- « mutation inactivant l'adhésine MIC3 » toute mutation résultant dans l'absence d'expression de MIC3, ou dans l'expression d'une protéine MIC3 non-fonctionnelle, c'est-à-dire ayant perdu sa fonction de liaison à la surface d'une cellule-hôte.

Des exemples de mutations résultant dans l'absence d'expression de MIC1 ou de MIC3 sont notamment la délétion de la totalité du gène correspondant, ou de sa région codante, ou de sa région promotrice. Des exemples de mutations résultant dans l'expression d'une protéine MIC3 non-fonctionnelle sont notamment des mutations affectant la région du gène *mic3* codant pour le domaine de type : « domaine de liaison à la chitine de la protéine MIC3 », à savoir les acides aminés 84-144 de ladite protéine. Il peut s'agir notamment de mutations affectant au moins le tryptophane en position 126 ou la phénylalanine en position 128 de la protéine MIC3.

Ces mutations peuvent être effectuées de manière classique par insertion, délétion, ou substitution d'une ou plusieurs bases au niveau de la séquence visée.

A titre d'exemples non-limitatifs de techniques de mutagenèse et de transformation utilisables chez les toxoplasmes, on citera notamment celles décrites dans les publications suivantes : KIM et al., (Science 262 : 911-914, 1993) ; DONALD et ROOS, (Mol. Biochem. Parasitol. 63 : 243-253, 1994) ; SOLDATI et al., (Mol. Biochem. Parasitol. 74 : 87-97, 1995) ; DONALD et ROOS, (Mol. Biochem. Parasitol. 91 : 295-305, 1998).

La présente invention a également pour objet l'utilisation d'une souche mutante *d'Apicomplexa,* et notamment de toxoplasme, conforme à l'invention, pour l'obtention d'un vaccin destiné à conférer une immunité protectrice contre une parasitose à *Apicomplexa,* en particulier contre la toxoplasmose.

La présente invention a aussi pour objet un vaccin caractérisé en ce qu'il comprend en tant que principe actif, une souche mutante *d'Apicomplexa* et notamment de toxoplasme, conforme à l'invention, telle que définie précédemment.

La présente invention sera mieux comprise à l'aide du complément de description qui va suivre, qui se réfère à des exemples de construction de mutants de *Toxoplasma gondii* dans lesquels MIC1 et/ou MIC3 sont inactivées, et d'utilisation vaccinale d'un double mutant dans lequel MIC1 et MIC3 sont inactivées.

Il doit être bien entendu toutefois que ces exemples sont donnés uniquement à titre d'illustration de l'objet de l'invention dont ils ne constituent en aucune manière une limitation.

### EXEMPLE 1 : INACTIVATION DE MIC1 ET/OU MIC3 CHEZ T. GONDII.

### Plasmides utilisés :

Les plasmides pmic3KO-1 et pmic3KO-2 ont été utilisés respectivement pour construire le simple mutant mic3KO à partir de la souche RHhxgprt⁻ et le double mutant mic1-3KO à partir de la souche mic1KO. Les plasmides pM3MIC3ty et pM2MIC1myc + pM3MIC3 été utilisés respectivement pour restaurer l'expression de MIC3 dans le mutant mic3KO (souche mic3KO+MIC3), et pour restaurer l'expression de MIC1 et MIC3 dans le double mutant mic1-3KO (souche mic1-3KO+MIC1-3)

### Plasmide pmic3KO-1

La région 3'UTR du gène *Mic3* (2136 pb) a été amplifiée par PCR à partir du plasmide pBlueMIC3 (CEREDE et al., 2002, précité), qui résulte de l'insertion d'un fragment d'ADN génomique de 2247 pb (GenBank AJ 132530) de *T.gondii* au site NotI du plasmide pBluescript II® SK(-).

Pour l'amplification, les amorces ML9: 5'-GTGTAAGCTTCAGCGAGTCTCTGAGAG-3' (SEQ ID NO: 1) et ML10: 5'-GGGGTACCGAGCTCATGAGCAGAAGCTGCCAG-3' (SEQ ID NO: 2) ont été utilisées. La zone amplifiée a été clonée entre les sites de restriction HindIII et KpnI du plasmide pminiHXGPRT (DONALD ET ROOS, 1998, précité). Un fragment d'ADN de 1977 pb de la région 5'UTR de *Mic3,* a été obtenu par digestion XbaI/NheI d'un fragment EcoRI de 3,5 kb de la séquence génomique en 5' de *Mic3,* et cloné au site XbaI de pminiHXGPRT.

Le plasmide résultant, qui contient le marqueur de sélection HXGPRT (hypoxanthine-xanthine-guanine phosphoribosyl transférase), encadré par les régions flanquant en 3' et 5' l'ORF de *mic3,* a été dénommé pmic3KO-1.

### Plasmide pmic3KO-2

Les régions 3'UTR et 5'UTR de *mic3,* obtenues comme décrit ci-dessus, ont été insérées dans le plasmide pTUB/CAT (KIM et al., 1993, précité), de part et d'autre de la séquence codant pour le marqueur de sélection chloramphénicol acétyl transférase (CAT), aux mêmes sites de restriction que ceux décrits pour le plasmide pmniHXGPRT.

Le plasmide résultant, contenant le marqueur de sélection CAT, encadré par les régions flanquant en 3' et 5' l'ORF de *mic3,* a été dénommé pmic3KO-2.

### Plasmide pM3MIC3ty

Ce plasmide a été construit à partir du plasmide pT8GFPPfmyoAtail (HETMANN et al., Mol. Biol. Cell 11 : 1385-1400, 2000), dans lequel a été ajouté un épitope TY (BASTIN et al., Mol. Biochem. Parasitol. 77(2) : 235-239, 1996) entre les sites NsiI et PacI. Ce plasmide contient le promoteur de la tubuline entre les sites KpnI et EcoRI et le gène codant pour la GFP bordé des sites EcoRI et NsiI.

La région du promoteur de *Mic3* (562 pb) a été amplifiée par PCR à partir du plasmide pBlueMIC3 avec les amorces ML23: 5'-CTGAATTCAGATCTTACCAGTGTTGGACAAGG-3' (SEQ ID NO : 3) et ML24 : 5'-GGGGTACCCCTTGCTAGGTAACCACTCGTGC-3' (SEQ ID NO : 4), et insérée à la place du promoteur de la tubuline aux sites KpnI et EcoRI.

L'amorce ML24 permet d'introduire un site de restriction BglII en amont du site EcoRI et de cloner ensuite le gène *Mic3* à la place du gène codant pour la GFP aux sites BglII et NsiI.

La séquence codant pour MIC3 a été amplifiée par PCR à partir du plasmide pBlueMIC3 avec les amorces ML11: 5'-GCACAATTGAGATCTAAAATGCGAGGCGGGACGTCC-3' (SEQ ID NO: 5) et ML15: 5'-TGCTATGCATTCCTAGGCTGCTTAATTTTCTCACACGTCAC-3' (SEQ ID NO : 6) introduisant respectivement les sites de restriction BglII et NsiI.

### Plasmides pM2MIC1myc et pM3MIC3

Le plasmide pM2MIClmyc (REISS et al., J. Cell Biol. 152 : 563-578, 2001) exprime la protéine MIC1, étiquetée par l'épitope myc à son extrémité C-terminale, sous le contrôle des séquences flanquantes en 5' et en 3' du gène *Mic2*

Le plasmide pM3MIC3 a été construit par clonage d'un fragment PvuI/SacI de 2072 pb du vecteur pBlueMIC3, contenant le gène *Mic3* et ses régions flanquantes en 5' et en 3', entre les sites Sacl et PacI du vecteur pT/230-TUB5/BLE (SOLDATI et al., Mol. Biochem. Parasitol. 74 : 87-97, 1995) contenant une cassette d'expression exprimant le marqueur de sélection à la phléomycine.

### Construction des souches mutantes mic3KO et mic1-3KO, et des souches mutantes complémentées mic3KO+MIC3, et mic1-3KO+MIC1-3

L'haploïdie du génome des Apicomplexes lors de la phase proliférative permet l'invalidation du gène *Mic3,* en une seule recombinaison homologue.

Tous les *T. gondii* utilisés ont été produits en fibroblastes humains (HFF) cultivés en milieu minimal de Dulbecco (DMEM) supplémenté par 10% de sérum de veau foetal (FCS), 2 mM glutamine, 50 U/ml de pénicilline et 50 µg/ml de streptomycine. Ils ont été récoltés lors de la lyse des cellules hôtes.

### Souche mic3KO

La souche de *T. gondii* utilisée pour la mutagenèse est la souche RHhxgprt⁻ (DONALD et ROOS, 1998, précité), déficiente pour le gène de l'hypoxanthine-xanthine-guanine phosphoribosyl transférase (HXGPRT), et de ce fait sensible à l'acide mycophénolique.

80-100 µg de plasmide pmic3KO-1 purifié puis linéarisé par KpnI ont été ajoutés à 10⁷ tachyzoïtes RHhxgprt⁻ mis en suspension dans du milieu d'électroporation CYTOMIX (VAN DEN HOFF et al., Nucleic Acids Res. 20 : 2902, 1992), et l'électroporation a été réalisée en cuvette d'écart 4 mm, dans un volume de 800 µl sur appareil BTX Electrocell Manipulator (Paramètres : 2 kV, R=48 ohms).

Après électroporation, les tachyzoïtes ont été déposés sur une monocouche de cellules HFF en culture. Pour la sélection des mutants, le lendemain de l'électroporation, le milieu de culture a été supplémenté par l'agent de sélection (25 µg/ml d'acide mycophénolique et 50 µg/ml de xanthine), et trois passages en culture sont effectués dans ce milieu.

Cinq jours après le dernier passage, les parasites sont clonés par dilution limite dans les puits d'une plaque à 96 puits de cellules HFF, en présence d'agent de sélection, et les clones retenus sont amplifiés.

### Souche mic1-3KO

La souche de *T. gondii* utilisée pour la mutagenèse est la souche miclKO (REISS et al., J. Cell Biol. 152 : 563-578, 2001) qui dérive de la souche RHhxgprt⁻ décrite ci-dessus par délétion du gène *Mic1* remplacé par le gène codant pour l'HXGPRT dans la souche mic1KO.

80-100 µg de plasmide pmic3KO-2 purifié puis linéarisé par KpnI ont été ajoutés à 10⁷ tachyzoïtes mic1KO pour une électroporation dans les conditions décrites ci-dessus.

Les mutants ont été sélectionnés et clonés comme décrit ci-dessus, en présence de 20 µM de chloramphénicol comme agent de sélection.

Les mutants dans lesquels MIC3 a été inactivée sont dénommés mic3KO, et ceux dans lesquels MIC1 et MIC3 ont été inactivées sont dénommés micl-3KO.

### Souche mic3KO+MIC3

L'expression de MIC3 a été restaurée par co-transfection des parasites mic3KO avec 100 µg du vecteur pM3MIC3ty, et 10 µg du plasmide pTUB/CAT.

La sélection a été effectuée en présence de 20 µM de chloramphénicol comme décrit ci-dessus.

La souche mic3KO complémentée par MIC3 est dénommée mic3KO+MIC3.

### Souche mic1-3KO+MIC1-3

L'expression de MIC1 et MIC3 a été restaurée par co-transfection des parasites micl-3KO avec 100 µg du vecteur pM2MIC1myc, et 10 µg du plasmide pM3MIC3.

Les mutants ont été sélectionnés et clonés comme décrit ci-dessus, en présence de 10 µg/ml de phléomycine comme agent de sélection.

La souche mic1-3KO complémentée par MIC1 et MIC3 est dénommée mic1-3KO+MIC1-3.

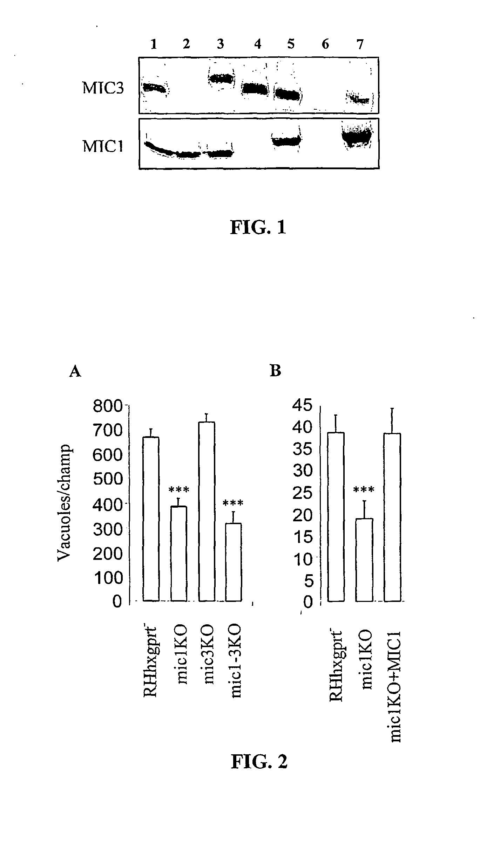

Les protéines totales des mutants mic3KO, mic1-3KO, mic3KO+MIC3, et mic1-3KO+MIC1-3 ont été analysées par électrophorèse SDS-PAGE et transfert de Western. Après extraction des protéines totales par ébullition des cellules dans du tampon SDS (en présence ou non de DTT 0,1 M) et séparation sur gels de polyacrylamide 10%, les protéines ont été transférées sur membrane de nitrocellulose. Les transferts de Western ont été marqués comme décrit par GARCIA-REGUET et al. (1998, précité) à l'aide d'anticorps monoclonaux anti-MIC3 (T42F3 au 1:400) et anti-MIC1 (T101F7) puis détectés par des IgG de chèvre anti-souris conjugués à la phosphatase alcaline (1 :1000). Les résultats sont présentés dans la Figure 1.

Légende de la Figure 1 :
1 = souche sauvage de *Toxoplasma gondii* RH
2 = mutant mic3KO
3 = mutant mic3KO+MIC3
4 = mutant mic1KO (REISS et al., 2001)
5 = mutant mic1KO+MIC1 (REISS et al., 2001)
6 = mutant mic1-3KO
7 = mutant mic1-3KO+MIC1-3

Les résultats montrent que l'expression de MIC3 est indétectable dans mic3KO (2) et micl-3KO (6) mais est restaurée dans mic3KO+MIC3 (3) et mic1-3KO+MIC1-3 (7). L'expression de MIC1 est indétectable dans mic1KO (4) et mic1-3KO (6) mais est restaurée dans mic1KO+MIC1 (5) mic1-3KO+MIC1-3 (7).

Ces résultats ont été confirmés par immunofluorescence. Les tachyzoïtes cultivés une nuit sur monocouche de cellules HFF, ont été lavés en PBS,et fixés par du formaldéhyde 4% pendant 20 min. Après 3 lavages, les cellules HFF infectées ont été perméabilisées avec 0, 1 % de Triton X-100 dans du PBS pendant 10 min, la réaction a été arrêtée par 10% de sérum de boeuf foetal (FBS) pendant 30 min., puis les cellules ont été incubées avec l'anticorps primaire (mAb anti-MIC1 TI01F7, mAb anti-MIC3 T42F3) dilué dans 2% FBS pendant 40 min., lavées et puis incubées avec un anticorps secondaire (chèvre anti-souris couplé au FITC, et rouge Texas chèvre anti-lapin). Les observations ont été réalisées sur un microscope Leica DMRA2 équipé pour l'épifluorescence et les images ont été enregistrées avec une caméra Princeton cooISNAP CCD.

Les résultats montrent qu'aucune protéine MIC3 n'est détectable dans des tachyzoïtes mic3KO, alors qu'une expression de MIC3de type sauvage est observée dans les micronèmes des souches complémentées (mic3KO+MIC3, mic1-3KO+MIC1-3). La complémentation de MIC1 dans mic1KO+MIC1 et mic1-3KO+MIC1-3 conduit à une certaine accumulation de MIC1 dans la vacuole parasitophore et dans l'espace périnucléaire.

### EXEMPLE 2 : EFFETS DE L'INACTIVATION DE MIC1 ET/OU MIC3 SUR LES PROPRIETES INFECTIEUSES DE T. GONDII

Les mutants de *Toxoplasma gondii* décrits à l'exemple 1 ci-dessus, ont été entretenus par passages réguliers sur cellules HFF cultivées en milieu DMEM supplémenté par 10% de sérum de veau foetal (FCS), 2 mM glutamine, 50 U/ml de pénicilline et 50 µg/ml de streptomycine.

### Capacité d'invasion

2x10⁵ tachyzoïtes de miclKO, miclKO+MIC1, mic3KO et micl-3KO purifiés ont été ajoutés à des cellules HFF cultivées sur des lamelles couvre-objet en verre. Les cellules ont été fixées pendant 12h puis colorées en utilisant un mélange bleu de méthylène-éosine (kit RAL 555), et montées sous lamelles, de façon permanente (PERTEX, Microm, France). Le nombre de vacuoles parasitaires, représentant la capacité d'invasion du parasite, a été compté dans 10 champs sélectionnés aléatoirement par lamelle, et les données ont été présentées comme la moyenne de vacuoles par champ provenant de 4 lamelles, sur la base de 5 répétitions indépendantes.

Le contrôle a été réalisé dans les mêmes conditions avec la souche RHhxgprt⁻, dont la capacité d'invasion est comparable à celle de la souche sauvage RH de *Toxoplasma gondii.*

Les résultats sont présentés dans la Figure 2 (*** = invasion significativement inférieure, p<0,001).

Les résultats montrent que la capacité d'invasion des parasites mic1KO est diminuée d'environ 50% par rapport à celle de la souche contrôle RHhxgprt⁻.

En revanche, la capacité d'invasion des parasites mic3KO et de ceux de la souche contrôle est comparable (Figure 2A). La capacité d'invasion des parasites micl-3KO n'est pas significativement différente de celle des mutants miclKO (Figure 2A), indiquant que MIC1 et MIC3 n'ont pas de fonction additive dans l'invasion des fibroblastes par *Toxoplasma gondii.*

La complémentation de mic1KO par MIC 1 restaure la capacité d'invasion à un niveau comparable de celui de la souche contrôle (Figure 2B).

### Virulence

Les souris meurent généralement 9 jours après une infection par voie intrapéritonéale par 20 tachyzoïtes de la souche sauvage RH de *Toxoplasma gondii.*

L'étude de la virulence des mutants mic1KO, mic3KO, micl-3KO, mic1KO+MIC1, mic3KO+MIC3 et mic1-3KO+MIC1-3 a été réalisée sur un lot de 10 souris mâles OF1 par injection intrapéritonéale de 20 tachyzoïtes/souris de la souche micl-3KO, et en suivant le devenir des souris infectées.

Les contrôles ont été réalisés dans les mêmes conditions sur un lot de 9 souris mâles OFI en utilisant la souche RHhxgprt⁻, dont la virulence est comparable à celle de la souche sauvage RH de *Toxoplasma gondi.*

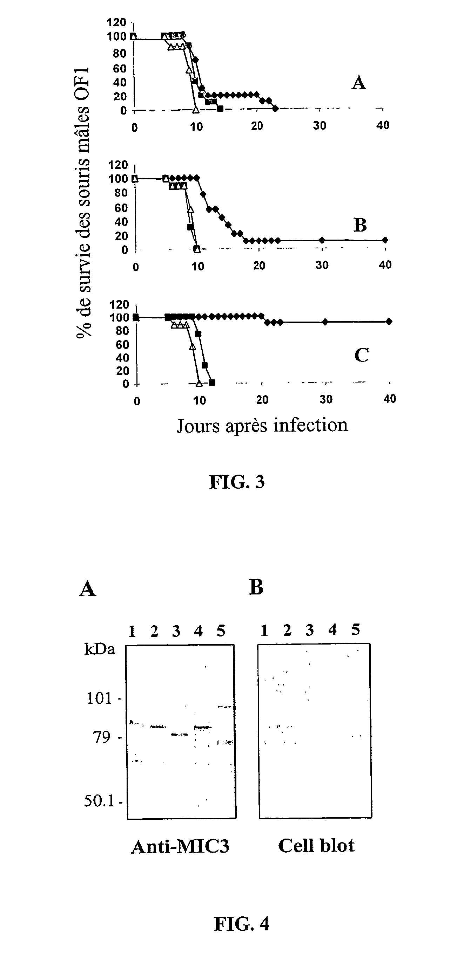

Les résultats sont représentés par la Figure 3.

Légende des Figures 3A, 3B et 3C :
◆ = souche mic1KO (A); souche mic3KO (B); souche mic1-3KO (C)
■ = souche mic1KO+MIC1 (A) ; souche mic3KO+MIC3; souche mic1-3KO+MIC1-3 (C)
△ = souche RHhxgprt⁻ (A, B et C)

Toutes les souris infectées par la souche RHhxgprt⁻ sont mortes 9 jours après l'infection. Les souris infectées par mic1KO ou mic3KO présentent un léger retard de la mortalité (mort des souris entre 9 et 22 jours après l'infection), qui n'est pas observé dans le cas des souris infectées par les mutants complémentés miclKO+MIC1 et mic3KO+MIC3. Une seule des souris infectée par mic3KO est demeurée en vie 44 jours après infection, et cet animal a développé une réponse anticorps spécifique de T. *gondii* (résultats non présentés). Ces résultats indiquent que l'inactivation isolée des gènes *Mic1* ou *Mic3* ne conduit qu'à une légère diminution de la virulence chez les souris.

En revanche, dans le cas des souris infectées par la souche micl-3KO, on observe une survie quasi totale (90% de survie 40 jours après l'infection). La complémentation de mic1-3KO par MIC1 et MIC3 (souche mic1-3KO+MIC1-3) restaure complètement la virulence des parasites.

Les souris ont également été infectées avec différentes quantités du double mutant micl-3KO et la dose létale (DL100) de micl-3KO a été comparée avec celle de la souche contrôle RHhxgprt⁻. Alors que la DL100 à 9 jours de la souche contrôle est inférieure à 20 tachyzoïtes, celle de la souche mic1-3KO est de l'ordre de 2x10³ tachyzoïtes.

### EXEMPLE 3: IMPLICATION DE LA FONCTION D'ADHESION DE MIC3 DANS LA VIRULENCE DE TOXOPLASMA GONDII

### Détermination de résidus impliqués dans la fonction d'adhésion de MIC3

Il a été montré (CEREDE et al., 2002, précité) que le domaine de type « domaine de liaison à la chitine », de MIC3 est essentiel pour la liaison à la surface de la cellule hôte.

Différentes mutations ont été introduites dans ce domaine, afin de déterminer les résidus essentiels à la fonctionnalité de MIC3.

Les mutations effectuées sont les suivantes :
- substitution de l'un des résidus cystéine aux positions 102 (mutant C102G), 107 (mutant C107G), 108 (mutant C108G), par un résidu glycine ;
- substitution du résidu proline en position 103 par un résidu alanine (mutant P 1 03A) ;
- substitution de l'un des résidus sérine aux positions 109 (mutant S109A) et 130 (mutant S130A) par un résidu alanine ;
- substitution de l'un des résidus tyrosine aux positions 96 (mutant Y96A), 135 (mutant Y135A), et 141 (mutant Y141A), par un résidu alanine ;
- substitution de l'un des résidus phénylalanine aux positions 97 (mutant F97A), 121 (mutant F121A), et 128 (mutant F128A), par un résidu alanine ;
- substitution du résidu tryptophane en position 126 par un résidu alanine (mutant W126A).

Les positions des mutations sont indiquées par référence à la séquence polypeptidique du précurseur de MIC3 (Genbank CAB56644).

Les mutations ont été effectuées par mutagenèse dirigée par PCR (Quickchange®, Stratagène), de la séquence codant pour la forme mature de MIC3, contenue dans le plasmide pOC2 (CEREDE et al., 2002, précité).

Les plasmides obtenus sont respectivement dénommés pC102 (mutant C102G), pC107 (mutant C107G), pC108 (mutant C108G), pP103 (mutant P103A), pS109 (mutant S109A), pS130 (mutant S130A), pY96 (mutant Y96A), pF97 (mutant F97A), pF121 (mutant F121A), pW126 (mutant W126A), pF128 (mutant F128A), pY135 (mutant Y135A), et pY141 (mutant Y141A).

Les plasmides ont été purifiés en utilisant le kit Qiagen kit® (Qiagen), et la présence des mutations attendues vérifiée par séquençage.

Les protéines MIC3 mutantes ont été exprimées par transfection de cellules BHK-21 (Baby Hamster Kidney, ATCC CCL-10) cultivées dans du milieu BHK-21 (Gibco-BRL) supplémenté avec du sérum de veau foetal (FCS) 5%, 2 mM de tryptose, 100 U/ml de pénicilline et 100 µg/ml de streptomycine.

Pour chaque plasmide, 3x10⁵ cellules BHK-21 préalablement cultivées sur des lamelles couvre-objet pendant 24h dans des plaques 24 puits, ont été transfectées avec le plasmide purifié, à l'aide de Lipofectamine®, selon les conditions préconisées par le fabricant (Gibco-BRL). Les cellules ont été cultivées 24h de plus avant l'analyse.

Les propriétés de liaison des protéines MIC3 mutantes ont été étudiées en analysant leur localisation dans les cellules BHk-21 transfectées. Les cellules BHK-21 transfectées ont été fixées par du paraformaldéhyde 3% dans du PBS pendant 15 min, puis lavées et perméabilisées avec 0,1% de Triton X-100 dans du PBS pendant 10 min. Les lamelles portant les cellules ont ensuite été lavées dans du PBS contenant 0,5% de SAB, et incubées pendant 1h dans le même tampon contenant un anticorps anti-MIC3 (T82C10, 1 :200) ou anti-V5 (1 :500), puis pendant 1h avec une IgG de chèvre conjuguée à la TRITC (Sigma, 1 :400), avec plusieurs lavages en PBS entre chaque incubation. Les lamelles ont ensuite été lavées et montées sur des lames de microscope. La visualisation a été effectuée à l'aide d'un microscope à épifluorescence.

Quatre catégories de mutants ont été définies selon leur localisation.

Dans la première catégorie (mutants C102G, C107G, C108G, Y141A, F121A), la protéine est retenue dans le système sécrétoire ; dans la seconde catégorie (Y135A, Y96A, F97A, S109A, P103A) la protéine est sécrétée normalement et se lie à la surface des cellules transfectées, comme le fait la protéine MIC3 mature de type sauvage ; dans la troisième catégorie, (W126A, Y128A) la protéine est sécrétée normalement mais ne se lie pas à la surface des cellules transfectées ; dans la quatrième catégorie (S130A), la protéine est sécrétée normalement et se lie à un réseau de matériel cellulaire déposé par les cellules à la surface des plaques de verre.

Ces résultats montrent que toutes les mutations affectant un résidu cystéine conduisent à un défaut majeur de sécrétion ; les protéines MIC3 mutantes correspondantes s'accumulent sous forme de larges vésicules périnucléaires, indiquant que ces protéines sont mal repliées ou assemblées de manière incomplète. Ce résultat est cohérent avec le rôle des cystéines dans le repliement du domaine.

En ce qui concerne les autres substitutions, les deux mutations F121A et Y141A affectent également la sortie des protéines. Les autres mutants (Y135A, Y96A, F97A, S109A, P103A, W126A, F128A, S130A) sont tous exprimés sous forme de dimère et sécrétés. Deux d'entre eux (W126A, Y128A) ne se lient pas aux cellules BHK-21 transfectées. Du fait de la perte de ces propriétés de liaison, ces deux mutants sont abondamment sécrétés dans le surnageant. Dans le cas du mutant S130A, le marquage par les anticorps anti-MIC3 n'est pas associé avec la membrane plasmatique de la cellule transfectée, comme dans le cas de la protéine MIC3 de type sauvage, mais avec l'espace intercellulaire, comme si la protéine R-MIC3 S130A était liée au matériel cellulaire déposé sur les plaques.

Ces résultats montrent que les deux résidus aromatiques W126 et Y128 sont impliqués dans l'interaction avec le récepteur de la surface de la cellule hôte, et que le résidu S 130 pourrait également contribuer aux propriétés de liaison de MIC3en participant à la spécificité d'interaction.

### Implication des résidus W126 et Y128 dans la virulence de T. gondii.

Pour étudier le rôle de la fonction d'adhésion de MIC3 dans la virulence de *T. gondii,* des expérimentations de complémentation de la souche double mutante micl-3KO par les mutants W126A et F128A ont été effectuées.

Les plasmides pM3MIC3W126A, pM3MIC3F128A, et pM3MIC3Y135A, ont été construits à partir du plasmide pM3MIC3. Ces plasmides portent le gène de sélection à la phléomycine, et expriment respectivement les mutants W126A, F128A, et Y135A. La présence des mutations attendues a été vérifiée par séquençage.

La souche mic1-3KO a été transfectée par les plasmides pM3MIC3W126A (souche mic1-3KO+MIC3W126A) ou pM3MIC3F128A (souche mic1-3KO+MIC3F128A). A titre de contrôles de liaison positifs la souche mic1-3KO+MIC3 et la souche mic1-3KO transfectée par le plasmide pM3MIC3Y135A (mic1-3KO+MIC3Y135A)ont été utilisées

L'expression des protéines MIC3 dans les différentes souches a été analysée par transfert de Western, et leurs propriétés de liaison ont été analysées par transfert de cellules. Le transfert de cellules a été réalisé avec un double de la feuille de nitrocellulose utilisée pour le transfert de Western, incubée avec une suspension de cellules Mode-K (VIDAL et al., J. Immunol. Methods 166 : 63-73, 1993) cultivées dans du milieu RPMI (Bio Whittaker) supplémenté avec 5% de sérum de veau foetal (FCS), 25 mM d'Hepes, 2 mM de glutamine, 100 U/ml de pénicilline et 100µg/ml de streptomycine.

Les résultats sont présentés dans les Figures 4A (transfert de Western) et 4B (transfert de cellules).

Légende des Figures 4A et 4B :
1 = souche sauvage de *Toxoplasma gondii* RH
2 = souche micl-3KO+MIC3
3 = souche micl-3KO+MIC3W126A
4 = souche micl-3KO+MIC3F128A
5 = souche micl-3KO+MIC3Y135A

Les résultats du transfert de Western montrent l'expression de protéines MIC3 dans toutes les souches. Toutes ces protéines migrent à la taille attendue pour un dimère en conditions réductrices. Cependant, les protéines W126A et Y135A ont migré plus vite que les autres, ce qui suggère une modification de conformation.

Les résultats du transfert de cellules montrent que comme attendu,les cellules se lient fortement à MIC3 native (1), MIC3myc (2), et à MIC3 Y135A (5). En revanche, les cellules sont incapables de se lier à MIC3 W126A (3) et MIC3 F128A (4). D'autre part, la modification de conformation de MIC3 Y135A n'affecte pas ses propriétés de liaison.

Les souches micl-3KO+MIC3W126A, micl-3KO+MIC3F128A, et à titre de contrôle, mic1-3KO+MIC3Y135A ont été utilisées pour analyser l'implication de la fonction d'adhésion de MIC3 dans la virulence chez la souris.

### Analyse de la virulence des souches

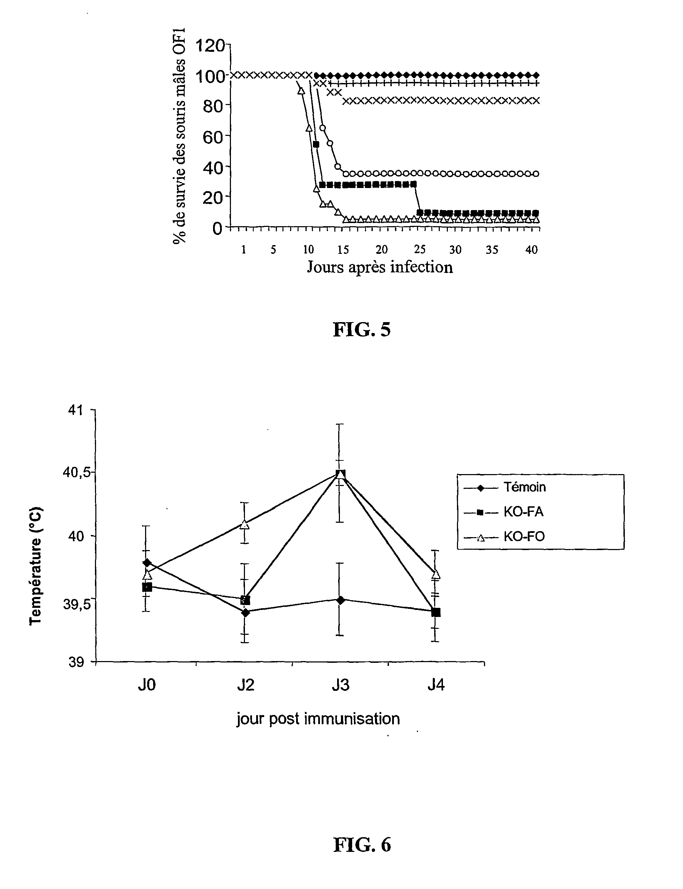

Le test de virulence a été réalisé comme décrit à l'exemple 2 : 20 tachyzoïtes de chaque parasite ont été injectées par voie intrapéritonéale dans des souris mâles OF1 (lot de 11 à 20 souris) dont la survie a été suivie pendant 40 jours. Les résultats sont présentés dans la Figure 5.

Légende de la Figure 5 :
◆ = souche mic1-3KO
■ = souche mic1KO
Δ = souche mic1-3KO+MIC3
○ = souche mic1-3KO+MIC3Y135A
+ = souche mic1-3KO+MIC3F128A
x = souche mic1-3KO+MIC3W126A

Comme attendu, les souris infectées avec mic1-3KO+MIC3 se comportent de la même façon que les souris infectées avec mic1KO, et meurent selon une cinétique similaire en 9-26 jours. La survie des souris infectées par la souche mic1-3KO+MIC3Y135A est supérieure (ce qui peut être dû à un adressage partiellement défectueux de la protéine MIC3 Y135A dans les vacuoles parasitophores).

En revanche, on observe, 40 jours après l'infection, une survie de respectivement 83,3% et 95% des souris infectées par la souche mic1-3KO+MIC3W126A et mic1-3KO+MIC3F128A.

Ces résultats montrent que la fonction de liaison de MIC3 aux cellules hôte est essentielle pour la virulence du parasite.

### EXEMPLE 4 : VACCCINATION DE LA SOURIS PAR LE MUTANT MIC1-3KO

### Protocole expérimental

Des lots de souris mâles OF1 âgées de 9,5 semaines, ont été traités comme suit :
- 21 souris (lot 1) ont reçu le mutant mic1-3KO ;
- 21 souris (lot 2) ont reçu le mutant mic1-3KO puis ont été réinfectées environ 1 mois plus tard par la souche de *Toxoplasma gondii* kystogène 76K ;
- 10 souris (lot 3) ont été infectées par la souche de *Toxoplasma gondii* kystogène 76K lors de la ré-infection du lot 2.

A J0, les souris des lots 1 et 2 ont reçu 20 tachyzoïtes du mutant micl-3KO par voie intrapéritonéale.

A J14, l'infection des souris a été contrôlée par recherche de la présence d'IgG anti-*Toxoplasma gondii,* à l'aide d'extrait total de toxoplasme (souche RH).

A J37, les souris effectivement immunisées (présence d'IgG anti-toxoplasmes) du lot 2 (8 souris), et du lot 3 (8 souris) ont été gavées par 70 kystes de la souche de *Toxoplasma gondii* 76K.

A J61, les souris des trois lots ont été sacrifiées. On a recherché la présence d'IgG anti-MIC3, due à l'infection par *Toxoplasma gondii* 76K, (puisque la souche vaccinale micl-3KO n'exprime pas cette protéine) et effectué un comptage des kystes cérébraux dans les broyats de cerveaux de ces souris (comptage sur cellule de Malassez ; la limite de détection est de 30 kystes par cerveau).

8 souris mâles OF1 naïves âgées de 15 semaines ont été gavées avec 1/3 du cerveau de chacune des 8 souris du lot 2 (contrôle de l'absence de parasites cérébraux). Une souris contrôle a reçu 60 kystes de la souche de *Toxoplasma gondii* 76K issus d'une souris du lot 3 (contrôle positif).

A J82, on a recherché la présence d'IgG anti-MIC3 chez les souris gavées à J61.

A J103, les souris ont été sacrifiées et un comptage des kystes cérébraux a été effectué.

### Résultats

### AJ14

Sur les 42 souris des lots 1 et 2, 8 sont mortes vers J10, lors de la phase aiguë d'infection (ce qui reflète la sensibilité élevée des souris à la virulence résiduelle du mutant MIC1-3KO). Le contrôle de l'infection par détection des IgG dirigés contre les antigènes parasitaires montre que 16 souris sont négatives et donc non infectées. Les lots 1 et 2 sont donc respectivement réduits à 10 et 8 souris.

### AJ61

### Recherche d'IgG anti-MIC3

Aucune IgG anti-MIC3 n'a été détectée dans le sérum des souris du lot 1 (contrôle négatif), alors que la présence d'IgG anti-MIC3 a été détectée dans le sérum des souris du lot 3 (contrôle positif). La présence d'IgG anti-MIC3 a été détectée dans le sérum des souris du lot 2, à l'exception d'une souris qui ne présente une très faible réponse.

### Comptage de kystes cérébraux

A l'examen microscopique,Les cerveaux des souris du lot 1 ne contiennent pas de kystes cérébraux (contrôle négatif), alors que cerveaux des souris du lot 3 contiennent de 2250 à 7250 kystes/cerveau soit une moyenne de 4037 kystes/cerveau (contrôle positif). Chez les souris du lot 2, 7 souris ne contiennent pas de kystes cérébraux et une souris contient 30 kystes cérébraux (soit un seul kyste observé sur 16 comptages de 10 µl sur cellule de Malassez).

### AJ82

Les souris naïves gavées avec les cerveaux des souris du lot 2 présentent des IgG anti-MIC3. La souris contrôle ayant reçu 60 kystes de la souche de *Toxoplasma gondii* 76K issus d'une souris du lot 3 présente également des IgG anti-MIC3.

### AJ103

Les souris naïves gavées avec les cerveaux des souris du lot 2 présentent respectivement 500, 375, 1000, 250, 165, 125, 310 et 375 kystes cérébraux. A titre de comparaison, la souris contrôle ayant reçu 60 kystes de la souche de *Toxoplasma gondii* 76K présente 2250 kystes cérébraux.

### Conclusion

Les souris immunisées par le mutant mic1-3KO ne forment pratiquement pas de kystes cérébraux lors d'une ré-infection avec la souche de *Toxoplasma gondii* 76K (protection de 99,9%).

En revanche, plusieurs des cerveaux de souris re-infectées sont infectieux par voie orale, donc l'immunisation par la souche vaccinale mic1-3KO n'est pas totalement stérilisante lors d'une ré-infection.

### EXEMPLE 5 : VACCCINATION DE BREBIS PAR LE MUTANT MIC1-3KO

### Animaux

Le statut immunitaire vis-à-vis de la toxoplasmose a été déterminé sur plus de soixante-dix brebis de race « pré-alpes du sud ». Seules trente-six brebis ont été retenues pour l'expérimentation pour leur séronégativité vis-à-vis de la toxoplasmose.

Les brebis sont maintenues tout au long de l'expérience en bergerie étanche sur le site de l'INRA de Nouzilly (Indre-et-Loire) afin de limiter au mieux les risques de contamination naturelle. Seuls le personnel animalier et les expérimentateurs, équipés de vêtements internes à la bergerie, peuvent pénétrer dans les bâtiments afin d'éviter la contamination du milieu et ne quittent la zone étanche qu'après s'être douchés. Les ustensiles utilisés et le matériel biologique ne sortent qu'après être passés dans un bain désinfectant et le lisier est incinéré.

Après vaccination, les brebis sont mises à la reproduction par saillie naturelle après synchronisation des chaleurs grâce à des éponges imprégnées d'hormones et placées dans le vagin des brebis.

### Souches de T gondii:

### Souche RH

La production d'extrait parasitaire total (ET) se fait à partir de la souche virulente RH (type I). Celle-ci est entretenue par passages successifs sur des souris OF1 femelles par injection intrapéritonéale de 10⁶ tachyzoïtes. Trois jours plus tard, les souris sont sacrifiées et les tachyzoïtes sont récupérés par lavage de la cavité intrapéritonéale avec 5 mL de milieu RPMI. Une numération des tachyzoïtes est effectuée sur cellule de Malassez, 10⁶ tachyzoïtes sont réinjectés par voie intrapéritonéale à des souris saines afin d'entretenir la souche.

Pour la préparation de l'extrait parasitaire total, les tachyzoïtes de la souche RH sont lavés, soniqués à 60 watt/s, trois fois durant 10 min et centrifugés à 2000 g pendant 30 min à 4°C. Le surnageant est concentré et aliquoté. La concentration est déterminée par un kit de dosage (Micro BGA) qui utilise comme standard de la BSA (Bovine Serum Albumin). Les aliquots sont conservés à -20°C.

### Souche vaccinale Mic1-3KO

La souche Mic1-3KO a été obtenue comme décrit à l'Exemple 1 ci-dessus. Elle est entretenue par passages successifs sur une lignée de fibroblastes de prépuce humain (HFF) cultivés dans du milieu IMDM auquel est ajouté du sérum de veau foetal 10 %, 50 mM de glutamate, 50 mM de pénicilline et 50 mM de streptomycine.

### Souche d'épreuve PRU

L'infection des brebis à mi-gestation est effectuée avec 400 oocystes de la souche PRU type II (souche Prugniaud) produits à partir de fécès purifiées de chats infectés.

### Protocole expérimental

### Vaccination

Les trente-six brebis séronégatives ont été divisées en trois lots de douze brebis :
- lot témoin
- lot qui reçoit 10⁵ tachyzoïtes Mic1-3KO, appelé « lot faible dose » (KO FA)
- lot qui reçoit 2.10⁶ tachyzoïtes Mic1-3KO, appelé « lot forte dose » (KO FO)

La vaccination de ces brebis a été effectuée par voie sous-cutanée.

### Relevés de température

Après l'immunisation, la température rectale des animaux a été relevée quotidiennement chaque matin jusqu'à ce qu'elle se stabilise à nouveau à des valeurs physiologiques. La température physiologique chez la brebis est de 38,5°C. Pour tenir compte de l'activité des animaux et du stress engendré par les manipulations, on considère qu'il y a hyperthermie à partir d'une température de 40,0°C.

### Etude de la réponse humorale

La réponse immunitaire humorale a été étudiée en évaluant par ELISA la cinétique d'apparition des IgG spécifiques anti-*T*. *gondii* dans le sérum.

### Obtention des sérums

Les sérums sont prélevés avant l'immunisation (J0) puis à J24, J39, J98, et J134 post-vaccination.

Le sang est prélevé au niveau de la veine jugulaire et le prélèvement (sans anticoagulant) est laissé une nuit à + 4°C pour permettre la formation du caillot. Le sérum est récupéré en centrifugeant les prélèvements à 1500 rpm pendant 5 min à + 20°C. Le surnageant est récupéré et aliquoté en échantillons de 3 mL et conservé à -20°C.

### Test ELISA

L'extrait total de la souche RH de *T. gondii,* obtenu comme décrit ci-dessus, est utilisé pour sensibiliser les puits à fond plat de plaques de microtitration (Nunc). 100 µL d'extrait (à une concentration de 10 µg/mL en tampon carbonate à 50 mM et pH = 9,6) sont déposés dans chaque puits. Après une nuit à + 4°C, trois lavages sont effectués en tampon PBS additionné de Tween-20 0,05 % (PBS-T) à l'aide d'un laveur automatique (MultiWash Advantage).

Les sites non spécifiques sont saturés par incubation des plaques pendant 1h 30 à 37°C atmosphère humide) avec du PBS à 4 % de BSA.

On dépose 100 µL de chaque échantillon de sérum dilué en PBS-T (dilutions au 1/50 et au 1/100), et on incube pendant 1h à 37°C.

Après deux séries de trois lavages, on dépose 100 µL d'anti-IgG de mouton couplé à la phosphatase alcaline (PAL ; Sigma), dilué au 1/5000 en PBS-T et on incube 1h30 à 37°C. Deux nouvelles séries de trois lavages sont effectuées et on effectue la révélation à l'aide de 100 µL de paranitrophénylphosphate (PNPP) à 1 mg/mL en DEA-HCl.

La lecture se fait après 10 à 20 minutes d'incubation, sur un lecteur de plaques (Wallac 1420 Multilabel counter) à la longueur d'onde λ = 405 nm.

Le seuil de positivité admis a été déterminé en fonction des valeurs d'absorbance (DO) des brebis du lot témoin : il est fixé à 0,35 de DO pour une dilution de sérum au 1/100.

### Infection

Une mise à la reproduction a été effectuée deux mois post-vaccination. A mi-gestation, l'infection des femelles gestantes a été réalisée par gavage avec 400 oocystes de la souche PRU. Pendant les jours suivant l'infection, la température rectale a été relevée quotidiennement, jusqu'au retour à des valeurs physiologiques et la réponse immunitaire humorale et cellulaire a été évaluée comme indiqué ci-dessus.

Enfin, les avortements fébriles et infectieux ont été relevés lors de la mise bas des brebis.

### RÉSULTATS

### Relevés des températures post-immunisation

Les moyennes des températures post-immunisation des brebis des différents lots sont représentées sur la Fiqure 6.

Les températures du lot témoin restent physiologiques. En revanche, un pic thermique à J3 est observé pour les deux lots vaccinés (40,5°C) ; l'hyperthermie du lot FO est plus précoce (40,1°C à J2) que celle du lot FA (39,5°C à J2). On observe un retour des températures à des valeurs physiologiques 4 jours après immunisation.

### Etude de la réponse humorale post-immunisation

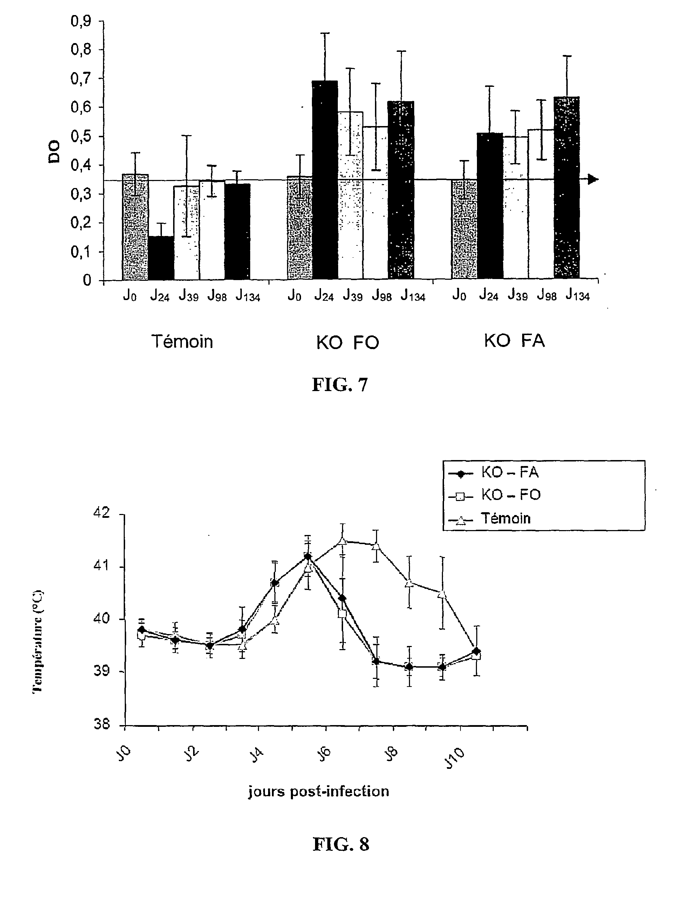

Les moyennes des résultats des tests ELISA à J0, J24, J39, J98, et J134 pour les sérums des différents lots de brebis dilués au 1/100 sont représentées sur la Figure 7.

Les brebis du lot témoin n'ont pas développé de réponse humorale.

Les brebis du lot FO et du lot FA ont développé une réponse IgG anti toxoplasmique dès J24. On observe un maintien de cette réponse jusqu'à la fin de l'expérimentation (J134).

### Rendement de gestation

Après la mise au mâle, sur les trente-six brebis de départ, seules trente étaient gestantes. Deux brebis non gestantes ont été observées dans chaque lot.

### Etude des relevés des températures post-infection.

Les moyennes des températures post-infection des brebis des différents lots sont représentées sur la Fiqure 8.

Suite à l'infection, une hyperthermie est observée chez l'ensemble des brebis.

Chez le lot témoin, le pic fébrile a duré cinq jours de J5 à J9 avec un maximum à 41,5°C à J6. Pour les deux lots vaccinés, le pic fébrile a duré trois jours de J4 à J6 avec un maximum à 41,2°C à J5.

Au moment du pic fébrile observé pour le lot témoin (J6), les températures des brebis des lots KO FA et KO FO ont déjà chuté (entre 40,0 et 40,5°C) alors que le pic observé chez les brebis du lot témoin se maintient jusqu'à J7 puis diminue progressivement jusqu'à J9 où la température moyenne observée est de 40,5°C (pour KO FA et KO FO de J7 à J9 : 39,0°C).

Le pic fébrile des brebis vaccinées est plus précoce, moins prolongé et avec un maximum fébrile plus faible que celui du lot témoin

### Suivi des avortements et des naissances

A la naissance, les agneaux morts ou viables, sont identifiés et pesés.

Il existe deux types d'avortements :
- les avortements fébriles dus à l'élévation de température suivant l'infection par les oocystes ;
- les avortements toxoplasmiques infectieux dus au développement du parasite chez le foetus.

### Avortements fébriles

Ces avortements interviennent consécutivement au pic thermique qui suit l'infection.

Les résultats sont résumés dans le Tableau I ci-dessous.

**Tableau I**

| Lot | Témoin | KO FA | KO FO |
|---|---|---|---|
| Avortements fébriles | 6/10 | 0/10 | 0/10 |

Six brebis du lot témoin ont avorté dans les quinze jours suivant l'infection. En revanche, aucun avortement fébrile n'a été observé chez les brebis vaccinées.

### Avortements dus à l'infection par le toxoplasme

Ces avortements interviennent au cours de la gestation, et sont dus au passage transplacentaire du parasite et à son développement chez le foetus. Ils sont généralement observés en fin de gestation.

Les résultats sont résumés dans le Tableau II ci-dessous.

**Tableau II**

| Lot | Témoin | KO FA | KO FO |
|---|---|---|---|
| Avortements infectieux | 4/10 | 1/10 | 3/9 |

Pour l'interprétation de ces résultats, une brebis du lot KO FO a été exclue : en effet dans le cas de cette brebis, la mise bas a été difficile : un des deux agneaux était viable. Mais ignoré par sa mère, il n'a pas survécu : il n'a donc pas été inclus dans les résultats.

Dans le lot témoin, aucune des quatre brebis qui n'avaient pas subi d'avortement fébrile n'a mené sa gestation à terme et donné naissance à un agneau viable.

Pour le lot faible dose (KO FA), une seule brebis sur dix a avorté.

Pour le lot forte dose (KO FO), trois avortements sur neuf brebis ont été enregistrés.

Le pourcentage de protection vaccinale globale a été évalué en fonction du nombre total d'avortements (fébriles + infectieux)). Lorsque la brebis a eu deux agneaux dont un vivant, l'avorton n'est pas considéré dans les résultats car plusieurs hypothèses sont envisageables pour expliquer cet avortement autres que l'infection : un manque de place pendant la gestation ou un manque d'intérêt et de soins pour son agneau juste après la naissance ; ces brebis sont donc comptabilisées comme protégées par la vaccination.

Les résultats sont résumés dans le Tableau III ci-dessous.

**Tableau III**

| Lot | témoin | Faible dose | Forte dose |
|---|---|---|---|
| Nombre d'avortements | 10 | 1 | 3 |
| Nombre total de brebis | 10 | 10 | 9 |
| Pourcentage de protection | 0% | 90% | 67% |

Le pourcentage de protection du lot KO FA est de 90 % contre 67 % pour le lot KO FO. Pour le lot témoin, la protection est nulle.

### Agneaux viables

Sept agneaux sont viables à la naissance pour neuf brebis gestantes dans le lot forte dose contre neuf agneaux viables pour dix brebis gestantes dans le lot faible dose. Ces agneaux sont en bonne santé et n'ont pas de malformation. Les poids s'échelonnent de 2200 à 5200 g et correspondent à des poids normaux d'un petit à terme (durée de gestation de cinq mois).

### EXEMPLE 6: COMPARAISON DE LA SOUCHE MIC1-3KO ET DE LA SOUCHE S48 (TOXOVAX®)

### Comparaison des profils immunoélectrophorétiques de tachyzoïtes mic1-3KO et de tachyzoïtes S48.

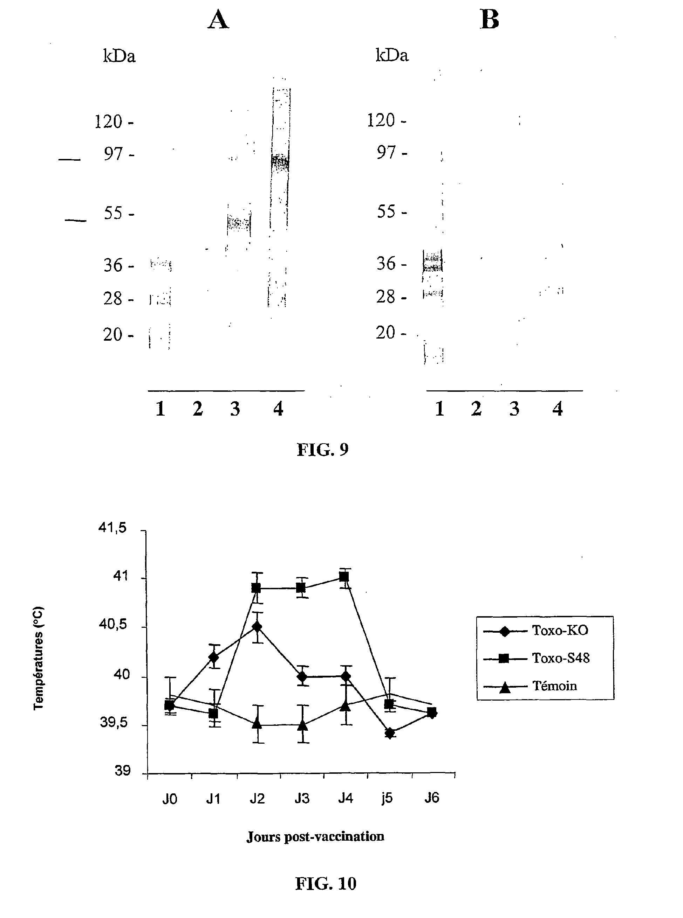

Les protéines totales de tachyzoïtes des souches mic1-3KO et S48 ont été analysées par électrophorèse SDS-PAGE en conditions non-réductrices et transfert de Western, comme décrit à l'exemple 1 ci-dessus. Les transferts de Western ont été révélés à l'aide d'anticorps monoclonaux anti-MIC3 (T82C10), ou anti-MIC1 (T101F7), d'un sérum de souris infectée par voie orale avec la souche 76K de *T. gondii,* ou d'un sérum de souris naïve. Les résultats sont présentés dans la Figure 9.

Légende de la Figure 9 : (A) lysat de tachyzoïtes S48 ; (B) lysat de tachyzoïtes mic1-3KO. Piste 1 : sérum anti-76 K ; piste 2 : sérum de souris naïve ; piste 3 : anti-MIC1 ; piste 4 : anti-MIC3.

Ces résultats montrent que les souches mic1-3KO et S48 ont des profils électrophorétiques très différents, et notamment que, contrairement à la souche mic1-3KO, la souche S48 exprime les deux protéines MIC1 et MIC3.

### Comparaison de la protection vaccinale conférée par les souches mic1-3KO et S48

La protection vaccinale conférée par l'une ou l'autre de ces souches a été évaluée en utilisant le protocole de l'exemple 5 ci-dessus.

Pour cela, un troupeau de brebis séronégatives a été divisé en trois lots:
- un lot témoin de 12 brebis.
- un lot (Toxo KO 1-3) de 13 brebis, immunisé avec 10⁵ tachyzoïtes de la souche mic1-3KO.
- un lot (Toxo S48) de 12 brebis, immunisé avec 10⁵ tachyzoïtes de la souche S48

Après inoculation des vaccins, les mesures suivantes ont été effectuées sur chacun des lots :
- relevé des températures post-immunisation
- suivi de la réponse humorale par technique ELISA
- infection par 400 oocystes de la souche PRU des femelles gestantes
- relevé des températures post-infection
- suivi des avortements et des naissances

### Températures post-immunisation

Les moyennes des températures post-immunisation des brebis des différents lots sont représentées sur la Figure 10.

### Réponse humorale post-immunisation

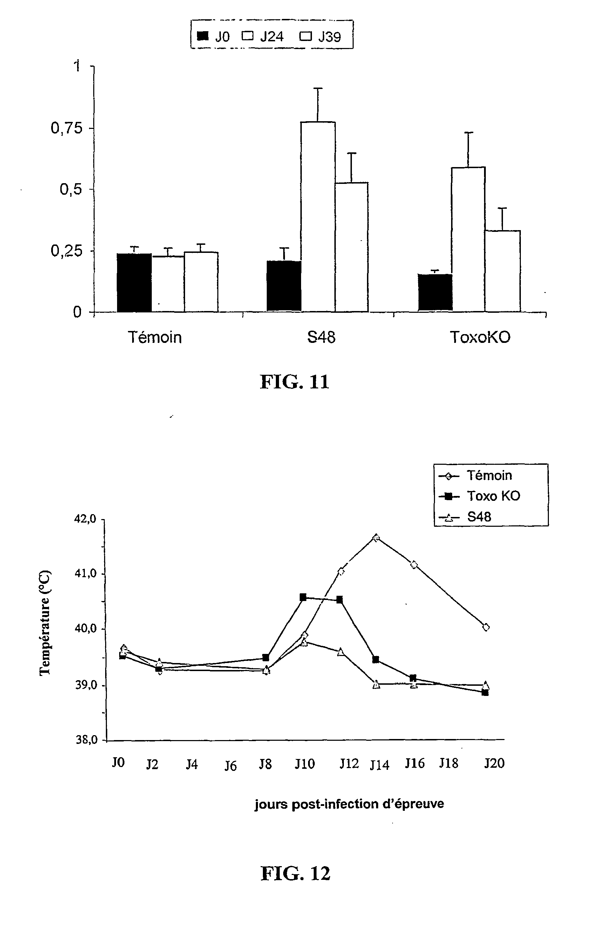

Les moyennes des résultats des tests ELISA à J0, J24, et J39, pour les sérums des différents lots de brebis dilués au 1/100, sont représentées sur la Figure 11.

### Rendement de gestation

Après la mise au mâle, 11 brebis témoin (sur les 12 de départ), 12 brebis ToxoKO (sur les 13 de départ), et 12 brebis Toxo S48 (sur les 12 de départ) étaient gestantes.

### Températures post-infection.

Les moyennes des températures post-infection des brebis des différents lots sont représentées sur la Figure 12.

### Suivi des avortements et des naissances

### Avortements fébriles (1 semaine après le pic thermique de l'infection d'épreuve)

Les résultats sont résumés dans le Tableau IV ci-dessous.

**Tableau IV**

| Lot | Témoins | ToxoKO | S48 |
|---|---|---|---|
| Avortements fébriles | 10/11 | 0/12 | 0/12 |

### Avortements infectieux (durant les 2 dernières semaines de gestation)

Les résultats sont résumés dans le Tableau V ci-dessous.

**Tableau V**

| Lot | Témoin | ToxoKO | S48 |
|---|---|---|---|
| Avortements infectieux | 1/11 | 4/12 | 4/12_ |

### Protection globale :

Les résultats sont résumés dans le Tableau VI ci-dessous.

**Tableau VI**

| Lot | Témoin | ToxoKO | S48 |
|---|---|---|---|
| Nombre d'avortements | 11 | 4 | 4 |
| Nombre total de brebis gestantes | 10 | 12 | 12 |
| Pourcentage de protection | 0% | 66,6% | 66,6% |

Le pourcentage de protection est de 66,6% pour chacun des lots ToxoKO et ToxoS48. Pour le lot témoin, la protection est nulle.

Les 2 souches MIC1-3KO et S48 confèrent donc un niveau de protection similaire.

## Revendications

1. Souche mutante d'un Apicomplexe de la famille des *Sarcocystidae* **caractérisée en ce qu'**elle comprend une mutation induisant l'absence d'expression de l'adhésine MIC1 et une mutation induisant l'absence d'expression de l'adhésine MIC3.

2. Souche mutante selon la revendication 1, **caractérisée en ce qu'**il s'agit d'une souche de toxoplasme.

3. Souche mutante selon la revendication 2, **caractérisée en ce qu'**il s'agit d'une souche de *Toxoplasma gondii.*

4. Utilisation d'une souche mutante selon une quelconque des revendications 1 à 3, pour l'obtention d'un vaccin.

5. Utilisation selon la revendication 4, **caractérisée en ce que** ledit vaccin est un vaccin anti-toxoplasmose.

6. Vaccin comprenant une souche mutante selon une quelconque des revendications 1 à 3.

## Claims

1. A mutant strain of an Apicomplex of the family *Sarcocystidae,* **characterized in that** it comprises a mutation inducing the absence of expression of the adhesin MIC1 and a mutation inducing the absence of expression of the adhesin MIC3.

2. The mutant strain as claimed in claim 1, **characterized in that** it is a strain of toxoplasma.

3. The mutant strain as claimed in claim 2, **characterized in that** it is a strain of *Toxoplasma gondii.*

4. The use of a mutant strain as claimed in any one of claims 1 to 3, for obtaining a vaccine.

5. The use as claimed in claim 4, **characterized in that** said vaccine is an anti-toxoplasmosis vaccine.

6. A vaccine comprising a mutant strain as claimed in any one of claims 1 to 3.

## Patentansprüche

1. Mutanter Stamm eines Apikomplexen der Familie *Sarcocystidae*, **dadurch gekennzeichnet, dass** er eine Mutation, die das Fehlen der Expression des Adhäsins MIC1 induziert, und eine Mutation, die das Fehlen der Expression des Adhäsins MIC3 induziert, umfasst.

2. Mutanter Stamm nach Anspruch 1, **dadurch gekennzeichnet, dass** es sich um einen Toxoplasmen-Stamm handelt.

3. Mutanter Stamm nach Anspruch 2, **dadurch gekennzeichnet, dass** es sich um einen Stamm von *Toxoplasma gondii* handelt.

4. Verwendung eines mutanten Stammes nach einem der Ansprüche 1 bis 3 zum Erhalt eines Impfstoffs.

5. Verwendung nach Anspruch 4, **dadurch gekennzeichnet, dass** der Impfstoff ein Anti-Toxoplasmose-Impfstoff ist.

6. Impfstoff, umfassend einen mutanten Stamm nach einem der Ansprüche 1 bis 3.
